# EUROPEAN PATENT APPLICATION

(11) **EP 4 781 999 A1**
(43) Date of publication of application: **29.07.2026**
(21) Application number: 25305087.6
(22) Date of filing: 24.01.2025
(51) Int. Cl.: A61K 38/17, A61P 21/00, A61P 25/00, G01N 33/68

(54) **ENGRAILED PROTEIN FOR USE IN THE TREATMENT OF TDP-43 PROTEINOPATHIES**

(71) Applicant: Brainever, 75012 Paris (FR); Institut National de la Santé et de la Recherche Médicale, 75013 Paris (FR); Centre National de la Recherche Scientifique, 75016 Paris (FR); College de France, 75005 Paris (FR)
(72) Inventor: The designation of the inventor has not yet been filed
(74) Representative: Gevers & Orès

(57) **Abstract**

The present invention relates to Engrailed protein, a nucleic acid encoding Engrailed protein and compositions comprising the same for use in treating or preventing diseases associated with accumulation of TDP-43 in the cell, particularly in the cell cytoplasm. Also provided herein are *in vitro* methods of selecting a subject for such therapy by detecting cellular accumulation of TDP-43, particularly cellular cytoplasmic accumulation, in a biological sample of the subject. Also provided herein are *in vitro* methods of following a subject treated with such therapy by measuring cellular accumulation, particularly cellular cytoplasmic accumulation, of TDP-43 in a biological sample of the treated subject.

## Description

The present invention relates to Engrailed protein, a nucleic acid encoding Engrailed protein and compositions comprising the same for use in treating or preventing diseases associated with accumulation of TDP-43 in the cell, particularly in the cell cytoplasm. Also provided herein are *in vitro* methods of selecting a subject for such therapy by detecting cellular accumulation of TDP-43, particularly cellular cytoplasmic accumulation, in a biological sample of the subject. Also provided herein are *in vitro* methods of following a subject treated with such therapy by measuring cellular accumulation, particularly cellular cytoplasmic accumulation, of TDP-43 in a biological sample of the treated subject.

Many neurodegenerative diseases are characterized by the deposition of insoluble protein in cells of the neuromuscular system. The transactive response (TAR)-DNA binding Protein TDP-43, also called TARDBP, is expressed at high-level during embryonic development, progressively decreases during postnatal development, and maintains at low level in adult neurons. High TDP-43 activity is essential for embryonic development but has been proven toxic for mature neurons. In various animal models (e.g. mouse, Wils et al., 2010, PNAS 107, 3858-3863) it has been shown that its overexpression leads to neurodegenerative diseases. Yang et al., 2022, PLoS One.;17(2):e0255710) have moreover suggested that persistent modest elevations in TDP-43 expression are enough to observe TDP-43 proteinopathy in several neurological disorders such as amyotrophic lateral sclerosis (ALS), Primary lateral sclerosis (PLS), inclusion body myopathy, Paget's disease and frontotemporal lobar degeneration [FTLD, including Frontotemporal dementia (FTD), Progressive Nonfluent Aphasia (PFNA), and Semantic Dementia (SD).

TDP-43 is a predominantly nuclear RNA binding protein, its function is essential for the survival of many types of cells in mammals; it is playing a critical role in fundamental RNA processing activities including RNA transcription, pre-mRNA splicing, RNA transport, as well as mRNA stability, and thus influences diverse cellular processes.

Nevertheless, TDP-43 protein has further been identified as a major component of the cytoplasmic inclusions (i.e. inclusion bodies) observed in neurons of subjects suffering of neurodegenerative diseases. It is the primary or secondary pathological hallmark of these diseases. More particularly, it was identified in about 50% of frontotemporal lobar degeneration (FTLD) cases and of 97% of amyotrophic lateral sclerosis (ALS) cases (Arai et al., 2006, Biochem Biophys Res Commun, 351:602-11; Scotter et al., 2015, Neurotherapeutics, 12:352-363). TDP-43 has also been shown to be a histopathological marker of multiple other neurodegenerative diseases, including Primary lateral sclerosis (PLS), inclusion body myopathy, Paget's disease, Frontotemporal dementia (FTD), Progressive Nonfluent Aphasia (PFNA), and Semantic Dementia (SD), Alzheimer's disease and hippocampal sclerosis (Amador-Ortiz et al., 2007, Ann Neurol., 61:435-45), Parkinson's disease and dementia with Lewy's bodies (Lin and Dickson, 2008, Acta Neuropathol., 116:205-13), and Huntington's disease (Schwab et al., 2008, J Neuropathol Exp Neurol., 67:1159-65). These diseases are collectively referred to as TDP-43 proteinopathies.

These neurodegenerative diseases despite different etiologies, clinical symptoms, and pathological hallmarks, demonstrate similar TDP-43 pathological manifestations in neurons including the elevation of TDP-43 expression and more particularly accumulation of ubiquitinated and/or hyper-phosphorylated TDP-43 insoluble aggregates in the cytoplasm, usually accompanied by the depletion of TDP-43 from the nucleus (Neumann et al., 2006, Science, 314:130-133). These nuclear clearance and cytoplasmic mislocalization with TDP-43 aggregation sequestering the normal function of TDP-43, actively block normal cellular processes and disrupt downstream processes including loss of TDP-43 nuclear RNA processing function, which destabilizes the transcriptome by multiple mechanisms including disruption of pre-mRNA splicing, the failure of repression of cryptic exons, and retrotransposon activation, trafficking of RNA granules, local translation within axons, and mitochondrial function, inevitably leading to cellular death and neurodegeneration. Nuclear clearance, cytoplasmic mislocalization and aggregation of TDP-43 are TAR DNA-binding protein 43 (TDP-43) toxicities, which lead to TDP-43 proteinopathies.

Several therapeutic approaches have been proposed to reduce onset or development of diseases associated with TDP-43 toxicity (Babazadeh et al.,2023, Ageing Research Reviews, 92, 2023,102085), such as for example :
Alquezar et al. (Molecular Neurodegeneration, 2016, 11:36) have investigated the effects of two brain penetrant CK-1δ inhibitors on TDP-43 phosphorylation as Casein kinase-1δ (CK-1δ) was reported to phosphorylate TDP-43.

Wang et al. (2012, Autophagy, 9(2), 239-240) have proposed to use autophagy activators (rapamycin, spermidine, carbamazepine and tamoxifen) for the treatment of TDP-43 related neurodegenerative diseases.

Commercially available monoclonal murine antibodies were primarily used in the studies on TDP-43 and to conduct pathological diagnosis of TDP-43 proteinopathies. Moreover, several patent applications such as WO2013/061163 or WO2022/034228 disclose anti-TDP-43 binding molecules, more particularly human anti-TDP-43 antibodies, for the treatment of TDP-43 pathologies.

Despite recent advances, therapeutic options for subjects with TDP-43-associated diseases are limited. Hence, there remains a genuine need to develop new and efficacious therapies for such TDP-43 proteinopathies. More particularly there is a need for means reducing amounts of cytoplasmic TDP-43, and more particularly amounts of cytoplasmic TDP-43 aggregates (i.e. TDP-43 inclusion bodies), in cells of the nervous system (e.g. motoneurons).

Unexpectedly, the Inventors have observed that administration of Engrailed protein is able to decrease TDP-43 expression levels in cells of the nervous system of a subject compared to TDP-43 expression levels in the subject before administration of the Engrailed protein.

The Inventors have further shown that administration of Engrailed protein is able to decrease cytoplasmic TDP-43 levels in cells of the nervous system of a subject compared to cytoplasmic TDP-43 levels in the subject before administration of the Engrailed protein.

More particularly, the Inventors have shown that administration of Engrailed protein is able to decrease cytoplasmic TDP-43 aggregates levels in cells of the nervous system of a subject compared to cytoplasmic TDP-43 aggregates levels in the subject before administration of the Engrailed protein.

An aspect of the invention thus relates to Engrailed protein, a nucleic acid encoding Engrailed protein, or a composition comprising said Engrailed protein or said nucleic acid for use in treating or preventing a disease associated with TDP-43 toxicity.

Another aspect of the invention relates to a method for treating or preventing a disease associated with TDP-43 toxicity by means of administration, to a patient in need thereof, of an effective amount of Engrailed protein, a nucleic acid encoding Engrailed protein or a composition comprising said Engrailed protein or said nucleic acid.

Another aspect of the invention further relates to the use of Engrailed protein, a nucleic acid encoding Engrailed protein or a composition comprising said Engrailed protein or said nucleic acid for the manufacture of a medicament for treating or preventing a disease associated with TDP-43 toxicity.

In some embodiments, the invention relates to a method of decreasing TDP-43 expression levels in a subject suffering from TDP-43 proteinopathy, comprising administration of the Engrailed protein or a nucleic acid encoding Engrailed protein, or a composition comprising the Engrailed protein or the nucleic acid encoding Engrailed protein, to the subject in an amount sufficient to decreasing TDP-43 expression levels in the subject compared to TDP-43 expression levels in the subject before administration of the Engrailed protein or the nucleic encoding Engrailed protein, or a composition comprising the Engrailed protein or the nucleic acid encoding Engrailed protein.

In some embodiments, the invention relates to a method of decreasing cytoplasmic TDP-43 levels in cells of the nervous system of a subject suffering from TDP-43 proteinopathy, comprising administration of the Engrailed protein or a nucleic acid encoding Engrailed protein, or a composition comprising the Engrailed protein or the nucleic acid encoding Engrailed protein, to the subject in an amount sufficient to decreasing cytoplasmic TDP-43 levels in cells of the nervous system of the subject compared to cytoplasmic TDP-43 levels in cells of the nervous system of the subject before administration of the Engrailed protein or the nucleic acid encoding Engrailed protein, or a composition comprising the Engrailed protein or the nucleic acid encoding Engrailed protein.

In some embodiments, the Invention relates to a method of decreasing cytoplasmic TDP-43 aggregates levels in cells of the nervous system of a subject suffering from TDP-43 proteinopathy, comprising administration of the Engrailed protein or a nucleic acid encoding Engrailed protein, or a composition comprising the Engrailed protein or the nucleic acid encoding Engrailed protein, to the subject in an amount sufficient to decreasing cytoplasmic TDP-43 aggregates levels in cells of the nervous system of the subject compared to cytoplasmic TDP-43 aggregates levels in cells of the nervous system of the subject before administration of the Engrailed protein or the nucleic acid encoding Engrailed protein, or a composition comprising the Engrailed protein or the nucleic acid encoding Engrailed protein.

In some embodiments, the Invention relates to a composition for use in the treatment or prevention of a disease associated with TAR DNA-binding protein 43 (TDP-43) toxicity, wherein the composition comprises a therapeutically effective amount of Engrailed protein or a nucleic acid encoding Engrailed protein, or a composition comprising the Engrailed protein or the nucleic acid encoding Engrailed protein.

In some embodiments, the Invention relates to a method of treating or preventing a disease associated with TAR DNA-binding protein 43 (TDP-43) toxicity in a subject in need thereof comprising administering a therapeutically effective amount of Engrailed protein or a nucleic acid encoding Engrailed protein in an amount effective to reduce TDP-43 aggregates in cells of the nervous system.

Another aspect of the Invention relates to a method comprising a step of determining the amount of TDP-43 in cells, particularly in the cytoplasm thereof, in a biological sample obtained from a subject to determine if an increased level of TDP-43 and/or an increased cytoplasmic accumulation is observed.

Another aspect of the Invention relates to the use of the amount of TDP-43 in cells, particularly in the cytoplasm thereof, in a biological sample obtained from a subject to determine if the subject can be selected for treatment with a composition comprising Engrailed protein or a nucleic acid encoding Engrailed protein.

In some embodiments, if an increased level of TDP-43 and/or an increased cytoplasmic accumulation of TDP-43, is observed, the subject can be selected for treatment with a composition comprising Engrailed protein or a nucleic acid encoding Engrailed protein.

In some embodiments, if the subject is selected for treatment with the composition, the subject is administered the composition comprising Engrailed protein or a nucleic acid encoding Engrailed protein.

In some embodiments, determining the amount of TDP-43 in cells of a biological sample, particularly in the cytoplasm of cells, comprises contacting the cells with an antibody specific for TDP-43.

According to another embodiment, the Invention relates to a method of selecting a subject for treatment with a composition comprising Engrailed protein or a nucleic acid encoding Engrailed protein.

In some embodiments, selecting a subject for said treatment comprises obtaining a biological sample from the subject and determining the amount of TDP-43 in cells of the biological sample, particularly in the cytoplasm of cells, and selecting the subject for said treatment.

In some embodiments, selecting a subject for said treatment comprises determining the amount of TDP-43 in cells of a biological sample from the subject, particularly in the cytoplasm of cells, and selecting the subject for said treatment.

Another aspect of the Invention relates to the use of cytoplasmic TDP-43 aggregates levels as biomarker to follow treatment efficacy of subject treated with Engrailed protein or a nucleic acid encoding Engrailed protein, or a composition comprising the Engrailed protein or the nucleic acid encoding Engrailed protein.

### Brief description of the drawings

The present disclosure will be more readily appreciated by reference to the following detailed description when being considered in connection with the accompanying drawings in which:
**Figure 1** shows comparative TDP-43 levels (nucleus and cytoplasm) in wild-type or En1-Het mice.
**Figure 2** shows levels of TDP43 in motoneurons of En1-Het mouse treated by EN1 injection.
**Figure 3** shows cytoplasmic TDP43 levels in motoneurons of En1-Het mouse treated by EN1 injection.

### Detailed description

As used herein, the term "subject" as used herein means a human or non-human animal selected for treatment.

The phrase "pharmaceutically-acceptable carrier" as used herein means a pharmaceutically-acceptable material, composition or vehicle, such as a liquid or solid filler, diluent, excipient, or solvent encapsulating material.

The phrases "therapeutically-effective amount" and "effective amount' as used herein means the amount of an agent which is effective for producing the desired therapeutic effect in at least a subpopulation of cells in a subject at a reasonable benefit/risk ratio applicable to any medical treatment.

"Treating' a disease in a subject or "treating' a subject having a disease refers to subjecting the subject to a pharmaceutical treatment, e.g., the administration of a drug, such that at least one symptom of the disease is decreased or prevented from worsening. This term encompasses inhibition, amelioration, prevention, or slowing of the disease or symptom of the disease.

As used herein, a therapeutic that "prevents" a disorder or condition refers to a compound that, when administered to a statistical sample prior to the onset of the disorder or condition, reduces the occurrence of the disorder or condition in the treated sample relative to an untreated control sample, or delays the onset or reduces the severity of one or more symptoms of the disorder or condition relative to the untreated control sample.

As used herein, a "TDP-43 proteinopathy" designates a disease (or disorder or condition) associated or characterized by the accumulation of TDP-43 in the cell, particularly in the cell cytoplasm and/or the mislocalization of nuclear TDP-43 to the cytoplasm of the cell. A TDP-43 proteinopathy may be characterized by aggregations of TDP-43 in a subject's central nervous system (CNS). TDP-43 proteinopathy are caused by TDP-43 toxicities.

Preferably, a TDP-43 proteinopathy according to the invention is selected among the list of diseases comprising - but not limited to - amyotrophic lateral sclerosis (ALS), frontotemporal lobar degeneration (FTLD), multiple system proteinopathy-A familial disorder in which patients present with ALS, FTLD, inclusion body myositis, Paget's disease of the bone or combinations of these phenotypes (MSP), Alzheimer's disease, dementia with Lewy bodies, Parkinson disease, Huntington disease, cerebral age-related TDP-43 with sclerosis (CARTS), limbic-predominant age-related TDP-43 encephalopathy (LATE), chronic traumatic encephalopathy (CTE), Perry disease, facial onset sensory and motor neuronopathy (FOSMN), sporadic inclusion body myositis (sIBM), primary progressive aphasia (PPA), corticobasal degeneration (CBD), primary progressive aphasia (PPA), hippocampal sclerosis and argyrophilic grain disease (AGD).

As is known to the skilled artisan, the "cytoplasm" includes the protoplasm of a cell excluding the nucleus and includes, but is not limited, to the cytosol and other cytoplasmic structures, e.g., inclusion bodies.

As used herein, the expressions "cytoplasmic inclusion body", "cytoplasmic TDP-43 aggregates" or similar term shall be taken to mean an abnormal structure that is detectable in cytoplasm comprising TDP-43 protein or variant thereof.

As used herein, a "nucleic acid encoding Engrailed protein" designates any polynucleotide that encodes an Engrailed protein. Preferably, a nucleic acid according to the invention comprises - but is not limited to - a gene encoding the Engrailed protein, preferably the human *En1* gene encoding EN1 Engrailed protein.

The Invention relates to the use of Engrailed protein or a nucleic acid encoding Engrailed protein for the preparation of a compositions for treating TDP-43 proteinopathy, and methods of using the same for the treatment of a disease or condition associated with TDP-43 toxicity.

The features disclosed herein for embodiments can thus apply *mutatis mutandis* to other aspects of the invention.

Provided herein are methods and compositions related to treating and/or preventing TDP-43 proteinopathies in a subject by obtaining a sample comprising cells, determining the relative level of TDP-43 (determining the amount of TDP-43) in the cells and/or cytoplasm of the cells in the sample and, if the subject has accumulation of TDP-43, particularly in the cell cytoplasm, administering to the subject Engrailed protein or a nucleic acid encoding Engrailed protein, or a composition comprising Engrailed protein or gene encoding Engrailed protein.

Also provided herein are methods of selecting a subject for therapy comprising administration of Engrailed protein or a nucleic acid encoding Engrailed protein, or a composition comprising Engrailed protein or a nucleic acid encoding Engrailed protein, the method comprising obtaining a sample of tissue comprising cells from the subject, determining the levels of TDP-43 (determining the amount of TDP-43) in the cells, in particular the cytoplasmic levels of TDP-43 in the cells, in the sample and, if TDP-43 has accumulated in the cells, particularly in the cytoplasm of the cells, and selecting the subject for therapy comprising administration of Engrailed protein or a nucleic acid encoding Engrailed protein, or a composition comprising Engrailed protein or a nucleic acid encoding Engrailed protein.

In some embodiments, the level (amount) of TDP-43 is determined for the full cell, the nuclear and/or cytoplasm of the cell.

The subject may be male or female. In some embodiments, the subject is an adult (i.e. 18 years of age or older). The subject may be a child (i.e. less than 18 years of age). In some embodiments, the subject is a mammal, preferably, a human. The subject may have a TDP-43 proteinopathy or may be at risk for a TDP-43 proteinopathy. For example, the subject may have a familial history of a TDP-43 proteinopathy.

As used herein, "biological sample" (or "sample") designates any liquid or tissue sample from a subject that may contain cells of the nervous system. It can be a biological fluid, such as blood, urine, saliva, cerebrospinal fluid, and the like. Within the brain, the tissue can be of the frontal cortex, temporal cortex, hippocampus, or brain stem, or a combination thereof. Preferably, a biological sample according to the invention may be cerebrospinal tissue, i.e. tissue located in or derived from the brain or spinal cord or a combination thereof. More preferably, if located in or derived from the spinal cord, the tissue may include or consist exclusively of cerebrospinal fluid (CSF).

In some embodiments, "cells" are cells of the nervous system, preferably neurons, and more preferably motoneurons.

In some embodiments, the methods disclosed herein comprise determining the level (amount) of TDP-43 in cells, particularly in the cytoplasm thereof, of a sample obtained from a subject (e.g. a cerebrospinal sample) to determine if an increased level of TDP-43 and/or an increased cytoplasmic accumulation is observed.

In some embodiments, if increased level of TDP-43 and/or increased cytoplasmic accumulation of TDP-43, is observed, the subject is selected for treatment with Engrailed protein or a nucleic acid encoding Engrailed protein, or a composition comprising Engrailed protein or the nucleic acid encoding Engrailed protein.

In some embodiments, if the subject is selected for the treatment, the subject is administered the Engrailed protein or a nucleic acid encoding Engrailed protein, or the composition comprising Engrailed protein or the nucleic acid encoding Engrailed protein.

Methods of determining the amount of TDP-43 within a sample can be carried on by contacting the sample, or isolated cells of the sample, with an antibody that binds to TDP-43 or a fragment thereof; and, determining the extent of binding of the antibody to the tissue and/or cells and/or part of the cells within the sample.

In some embodiments, analyzing the full cell, the nuclear and/or cytoplasmic levels of TDP-43 comprises contacting sample, or isolated cells of the sample, with an antibody specific for TDP-43. The antibody may have a detectable label (i.e. a fluorescent label). Antibody binding may be determined, for example, through microscopy or any other method known in the art. In some embodiments, the protein from the cells within the sample may be isolated and contacted with a TDP-43 antibody. The TDP-43 antibody may be immobilized on a solid support. In some embodiments, the antibody is specific for wild-type TDP-43. In other embodiments, the TDP-43 antibody specifically binds a specific form of TDP-43, such as phosphorylated or ubiquitinated form of TDP-43. In some embodiments, the antibody is polyclonal. In some embodiments, the antibody is monoclonal. In some embodiments, the TDP-43 antibody can be of any species. In some embodiments, the antibody is a mouse, rat, sheep, goat, camel, chicken, duck, hamster, guinea pig, dog, monkey, human or synthetic antibody or a combination thereof.

In some embodiments, TDP-43 is accumulated in the cytoplasm if at least 1%, at least 5%, at least 10%, at least 20%, at least 30%, at least 40%, at least 50% , at least 60% or at least 70% of total TDP-43 in the cells of the sample is in the cytoplasm of the cells.

In other embodiments, TDP-43 is accumulated in the cytoplasm if the amount of TDP-43 in the cytoplasm in the cells of the sample from the examined subject is greater than a sample from a healthy subject.

The methods disclosed herein comprise determining whether TDP-43 is accumulated in the full cell, in the cell nucleus and/or in the cell cytoplasm. In preferred embodiments, the methods disclosed herein comprise determining whether TDP-43 is accumulated in the cell cytoplasm. In some embodiments, TDP-43 is accumulated in the cytoplasm if at least 1%, at least 10%, at least 20%, at least 30%, at least 40%, at least 50%, at least 60% or at least 70% of total TDP-43 in the cells in the sample is localized in the cytoplasm of the cells.

In some embodiments, TDP-43 is accumulated in the cytoplasm if at least 1%, at least 5%, at least 10%, at least 20%, at least 30%, at least 40%, at least 50%, at least 60% or at least 70% of the cells in the sample have TDP-43 localized to the cytoplasm of the cell.

In some embodiments, TDP-43 is accumulated in the cytoplasm of the cells within the sample if the amount of TDP-43 in the cytoplasm of the cells within the sample is greater than the amount of TDP-43 in the cytoplasm of cells in a sample from a healthy subject (e.g. a subject that does not have a TDP-43 proteinopathy). In some embodiments, a reference (e.g. a sample from a healthy subject) may be used to compare the amount and localization of TDP-43 obtained in the methods disclosed herein from a sample (e.g. a cerebrospinal sample). The reference may be obtained on the basis of one or more samples from normal or healthy subject, in particular samples which are not affected by TDP-43 proteinopathy, either obtained from a subject or one or more different subjects. A reference may be determined empirically by testing a sufficiently large number of normal samples. A reference may also include immortalized cell lines.

In some embodiments, the phosphorylation status of TDP-43 is determined. In some embodiments, at least 1%, at least 5%, at least 10%, at least 20%, at least 30%, at least 40%, at least 50%, at least 60% or at least 70% of the TDP-43 in the cells of a sample are phosphorylated.

In some embodiments, the ubiquitination status of TDP-43 is determined. In some embodiments, at least 1%, at least 5%, at least 10%, at least 20%, at least 30%, at least 40%, at least 50%, at least 60% or at least 70% of the TDP-43 in the cells of a sample are ubiquitinated.

Exemplary methods for determining the phosphorylation or ubiquitination state of TDP-43 may be determined by any method known in the art, including but not limited to kinase activity assays, phospho-antibodies, western blot, or ELISA.

In some embodiments, the Invention relates to methods of selecting a subject for treatment with Engrailed protein or a nucleic acid encoding Engrailed protein, or a composition comprising Engrailed protein or the nucleic acid encoding Engrailed protein. In some embodiments, selecting a subject for therapy comprises obtaining a sample (e.g. a cerebrospinal sample) comprising cells from the subject and determining the amount of TDP-43 in cells of a sample, particularly in the cytoplasm of cells, and selecting the subject for therapy comprising administration of Engrailed protein or the nucleic acid encoding Engrailed protein, or a composition comprising Engrailed protein or the nucleic acid encoding Engrailed protein. In some embodiments, the subject is selected if cytoplasmic accumulation of TDP-43 is at least 1%, at least 5%, at least 10%, at least 20%, at least 30%, at least 40%, at least 50%, at least 60% or at least 70% of total TDP-43 in the cells of the sample. In other embodiment, the subject is selected if the amount of TDP-43 in the cytoplasm in the cells of the sample from the examined subject is greater than a sample from a healthy subject.

In some embodiments, the Invention relates to a methods of decreasing TDP-43 levels in cells of the nervous system of a subject suffering from TDP-43 proteinopathy, comprising administration of the Engrailed protein or a nucleic acid encoding Engrailed protein, or a composition comprising the Engrailed protein or the nucleic acid encoding Engrailed protein, to the subject in an amount sufficient to decrease TDP-43 levels in cells of the nervous system of the subject compared to TDP-43 expression levels in cells of the nervous system of the subject before administration of the Engrailed protein or the nucleic acid encoding Engrailed protein, or a composition comprising the Engrailed protein or the nucleic acid encoding Engrailed protein.

In some embodiments, the Invention relates to methods of decreasing cytoplasmic TDP-43 levels in cells of the nervous system of a subject suffering from TDP-43 proteinopathy, comprising administration of the Engrailed protein or a nucleic acid encoding Engrailed protein, or a composition comprising the Engrailed protein or the nucleic acid encoding Engrailed protein, to the subject in an amount sufficient to decrease cytoplasmic TDP-43 levels in cells of the nervous system of the subject compared to cytoplasmic TDP-43 levels in cells of the nervous system of the subject before administration of the Engrailed protein or the nucleic acid encoding Engrailed protein, or a composition comprising the Engrailed protein or the nucleic acid encoding Engrailed protein.

In some embodiments, the Invention relates to methods of decreasing cytoplasmic TDP-43 aggregates levels in cells of the nervous system of a subject suffering from TDP-43 proteinopathy, comprising administration of the Engrailed protein or a nucleic acid encoding Engrailed protein, or a composition comprising the Engrailed protein or the nucleic acid encoding Engrailed protein, to the subject in an amount sufficient to decrease cytoplasmic TDP-43 aggregates levels in cells of the nervous system of the subject compared to cytoplasmic TDP-43 aggregates levels in cells of the nervous system of the subject before administration of the Engrailed protein or the nucleic acid encoding Engrailed protein, or a composition comprising the Engrailed protein or the nucleic acid encoding Engrailed protein.

In some embodiments, the Invention relates to methods of decreasing TDP-43 gene expression levels in cells of the nervous system of a subject suffering from TDP-43 proteinopathy, comprising administration of the Engrailed protein or a nucleic acid encoding Engrailed protein, or a composition comprising the Engrailed protein or the nucleic acid encoding Engrailed protein, to the subject in an amount sufficient to decrease TDP-43 gene expression levels in cells of the nervous system of the subject compared to TDP-43 expression levels in the subject before administration of the Engrailed protein or the nucleic acid encoding Engrailed protein, or a composition comprising the Engrailed protein or thenucleic acid encoding Engrailed protein.

In some embodiments, the Invention relates to a composition for use in the treatment of a disease or condition associated with TAR DNA-binding protein 43 (TDP-43) toxicity, wherein the composition comprises a therapeutically effective amount of Engrailed protein or a nucleic acid encoding Engrailed protein, or a composition comprising the Engrailed protein or the nucleic acid encoding Engrailed protein.

In some embodiments, the Invention relates to methods of treating a disease or condition associated with TAR DNA-binding protein 43 (TDP-43) toxicity in a subject in need thereof comprising administering a therapeutically effective amount of Engrailed protein or a nucleic acid encoding Engrailed protein in an amount effective to reduce TDP-43 aggregates in the cytoplasm of cells of the nervous system.

In some preferred embodiments, the subject suffering from TDP-43 proteinopathy is a subject suffering from neurodegenerative disease with overexpression of TDP-43, particularly ubiquitinated and/or hyper-phosphorylated TDP-43 overexpression in cells of the nervous system and/or a subject suffering from neurodegenerative disease with cytoplasmic TDP-43 aggregates, particularly cytoplasmic ubiquitinated and/or hyper-phosphorylated TDP-43 aggregates in cells of the nervous system.

In some embodiments, therapeutically effective amount of Engrailed protein or the nucleic acid encoding Engrailed protein is an amount effective to reduce TDP-43 levels in the cells of the nervous system, preferably in the cytoplasm of cells of the nervous system.

In some embodiments, therapeutically effective amount of Engrailed protein or the nucleic acid encoding Engrailed protein is an amount effective to reduce TDP-43 aggregates in the cytoplasm of cells of the nervous system.

In some preferred embodiments, cells of the nervous system are neurons, and more particularly motoneurons.

In some embodiments, the TDP-43 proteinopathy is selected in the group comprising amyotrophic lateral sclerosis (ALS), frontotemporal lobar degeneration (FTLD), multiple system proteinopathy-A familial disorder in which patients present with ALS, FTLD, inclusion body myositis, Paget's disease of the bone or combinations of these phenotypes (MSP), Alzheimer's disease, dementia with Lewy bodies, Parkinson disease, Huntington disease, cerebral age-related TDP-43 with sclerosis (CARTS), limbic-predominant age-related TDP-43 encephalopathy (LATE), chronic traumatic encephalopathy (CTE), Perry disease, facial onset sensory and motor neuronopathy (FOSMN), sporadic inclusion body myositis (sIBM), primary progressive aphasia (PPA), corticobasal degeneration (CBD), primary progressive aphasia (PPA), hippocampal sclerosis and argyrophilic grain disease (AGD).

In some preferred embodiments, the TDP-43 proteinopathy is selected in the group consisting of frontotemporal lobar degeneration (FTLD), amyotrophic lateral sclerosis (ALS), Alzheimer's disease, hippocampal sclerosis, Parkinson's disease, dementia with Lewy's bodies and Huntington's disease.

As used herein, the term Engrailed protein relates to Engrailed 1 (EN1) from a mammal such as a primate, human, monkey, rabbit, pig, bovine, or rodent, and biologically active derivatives thereof. Mutant and variant Engrailed protein having activity are also embraced, as are functional fragments and fusion proteins of the EN protein. Exemplary EN1 protein or polypeptide includes, without limitation, human EN1 protein or polypeptide having primary amino acid sequence as annotated under Genbank accession number AAA 53502.2 or NCBI NP_001417.3. In certain embodiments, the Engrailed protein is a human Engrailed or recombinant human Engrailed protein, or a biologically active derivative or fragment thereof.

As used herein, the term "biologically active derivative" refers to any polypeptide with substantially the same biological function as Engrailed. The polypeptide sequences of the biologically active derivatives may comprise deletions, additions and/or substitution of one or more amino acids whose absence, presence and/or substitution, respectively, do not have any substantial negative impact on the biological activity of polypeptide.

As used herein, the terms "Engrailed" and "biologically active derivative", respectively, also include polypeptides obtained via recombinant DNA technology or synthetically. The recombinant Engrailed, e.g. recombinant human Engrailed may be produced by any method known in the art. This may include any method known in the art for (i) the production of recombinant DNA by genetic engineering, e.g. via reverse transcription of RNA and/or amplification of DNA, (ii) introducing recombinant DNA into prokaryotic or eukaryotic cells by transfection, i.e. via electroporation or microinjection, (iii) cultivating said transformed cells, e.g. in a continuous or batchwise manner, (iv) expressing Engrailed, e.g. constitutively or upon induction, and (v) isolating said Engrailed, e.g. from the culture medium or by harvesting the transformed cells, in order to (vi) obtain substantially purified recombinant Engrailed, e.g. via anion exchange chromatography or affinity chromatography.

Alternatively, the present Invention relates to the use of a recombinant expression system or recombinant vector encoding Engrailed protein (i.e. a nucleic acid encoding Engrailed protein) instead of the protein or peptide itself. As used herein, the term "recombinant expression system" or "recombinant vector" refers to a genetic construct or constructs for the expression of certain genetic material formed by recombination. The term "vector" as used herein refers to a nucleic acid molecule or a viral particle that contains elements necessary to allow delivery, propagation and/or expression of one or more nucleic acid molecule(s) within a host cell or organism. According to preferred embodiment, the genetic transfer is mediated by a DNA viral vector, such as an adenovirus (Ad) or adeno-associated virus (AAV); preferably an AAV. Non-limiting exemplary serotypes useful in the methods disclosed herein include any of existing serotypes, e.g., AAV2, AAV8, AAV9, or variant serotypes. In preferred embodiments, the AAV refers to the serotype AAV2 and variants thereof.

In some embodiments, the compositions which comprise a therapeutically-effective amount of Engrailed protein or a nucleic acid encoding Engrailed protein are formulated with one or more pharmaceutically acceptable carriers and/or diluents.

In some embodiments, the pharmaceutical compositions described herein may be specially formulated for intrathecal administration.

In some embodiments, the composition comprises additional agents. In some embodiments, the composition comprises an effective amount of Engrailed protein, a stabilizing concentration of reduced glutathione, an osmotic adjusting agent, and a buffering agent providing a pH less than 7, preferably less than 6, more preferably less than 5 and advantageously of about 4 to the formulation.

In some embodiments, the composition comprising Engrailed protein comprises:
- effective amount of Engrailed protein;
- from about 10 µM to about 100 µM reduced glutathione;
- from about 240 to about 290mM of an osmotic adjusting agent, preferably dextrose ;
- from about 0.1 mM to about 3 mM of MgCl₂;
- from 0 to 0.01 % polysorbate 20; and
- a buffering agent for maintaining pH at about 4.

In some embodiments, effective amount of Engrailed protein is selected from about 0.05 mg/mL and about 1 mg/mL, from about 0.1 mg/mL and about 0,9 mg/mL, from about 0.05 mg/mL and about 0,9 mg/mL, or is about 0.6 mg/mL.

In some embodiments, the composition comprising Engrailed protein comprises:
- about 0.6mg/ml of EN protein;
- about 250 mM of dextrose;
- about 2.13 mM of MgCl₂;
- about 50 µM of reduced glutathione;
- about 0.005% polysorbate 20 ; and
- about 5mM sodium acetate/acetic acid for maintaining a pH about 4.

The formulations of the Engrailed protein may be presented in unit dosage form and may be prepared by any methods well known in the art of pharmacy.

The amount of active ingredient (Engrailed protein or a nucleic acid encoding Engrailed protein) which can be combined with a carrier material to produce a single dosage form will vary depending upon the subject being treated and the particular mode of administration. The amount of active ingredient which can be combined with a carrier material to produce a single dosage form will generally be that amount of the agent which produces a therapeutic effect. The active ingredient can also be in microencapsulated form.

Actual dosage levels and administration regimen of the compositions disclosed herein may be varied so as to achieve the desired therapeutic response for a particular subject and mode of administration.

In some embodiments, administration of the composition comprises administration of the composition in one or more dose(s). In some embodiments, administration of the composition comprises administration of the composition in one or more, five or more, ten or more, twenty or more, thirty or more, forty or more, fifty or more, one hundred or more, or one thousand or more dose(s).

The exact dose will depend on the purpose of the treatment and will be ascertainable by one skilled in the art using known techniques. Doses ranging from 0.01 mg to 18 mg of Engrailed protein may be administered as intrathecal or intracerebral administrations.

The preferred delivery route is intrathecal or intracerebroventricular administration, more preferred delivery route is intrathecal. The volume of intrathecal injections ranges from 0.1 to 20 mL.

The compositions may be administered over any period of time effective to achieve the desired therapeutic response for a particular subject and mode of administration, without being toxic to the subject. The period of time may be at least 1 day, at least 10 days, at least 20 days, at least 30, days, at least 60 days, at least three months, at least six months, at least a year, at least three years, at least five years, or at least ten years. The dose may be administered when needed, sporadically, or at regular intervals. For example, the dose may be administered monthly, weekly, biweekly, triweekly, once a day, or twice a day.

The methods described herein may be performed or administered conjointly with any other treatment for TDP-43 proteinopathies.

According to another embodiment, the Invention relates to the use of cytoplasmic TDP-43 aggregates levels as biomarker to follow treatment efficacy of subject treated with Engrailed protein or a nucleic acid encoding Engrailed protein, or a composition comprising the Engrailed protein or the nucleic acid encoding Engrailed protein.

### EXAMPLES

### 1. Comparative TDP-43 levels (nucleus and cytoplasm) in wild-type or En1-Het mice

Total TDP-43 levels (nucleus and cytoplasm) in motoneurons have been compared in 4.5 months old WT or En1^{+/-} (En1-Het mice; Sonnier et al., 2007, Journal of Neuroscience, 27, 1063-1071) mice.

TDP-43 protein levels and localization have been determined using pan-TDP43 antibody. TDP-43 nuclear or cytoplasmic localization of TDP-43 has been determined by confocal microscopy (images are not shown).

TDP-43 levels have been measured in all motoneurons (MNs), in αMNs or in γMNs.

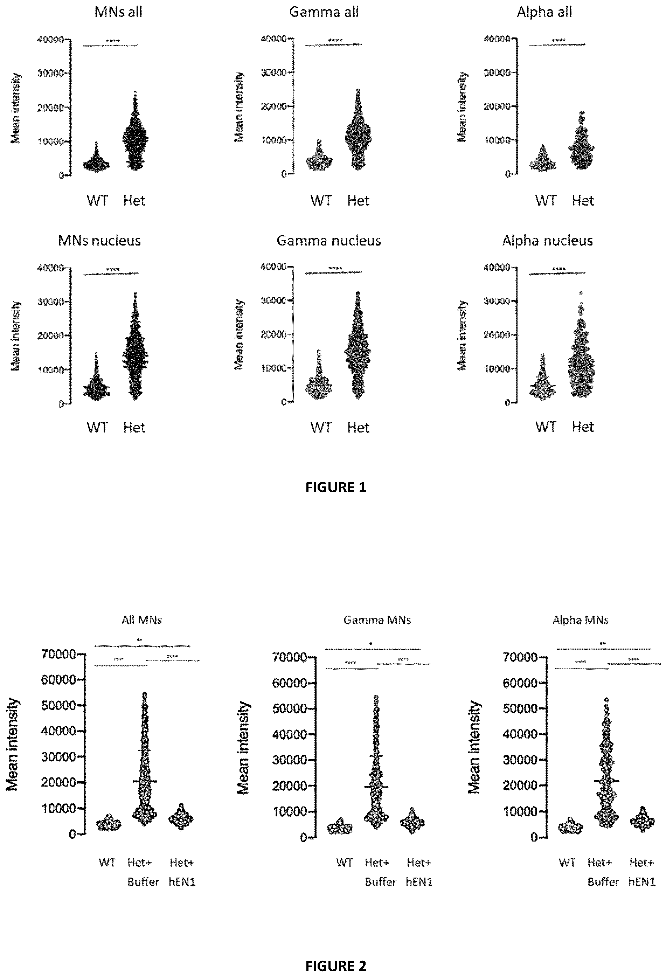

Results presented in Figure 1 showed :
- In WT mice, TDP43 is either poorly expressed and is present exclusively in the nucleus (not shown)
- In En1-Het mice, TDP43 is upregulated and is present both in nucleus and cytoplasm. Cytoplasmic TDP43 is in aggregates.

### 2. Engrailed 1 effect has been evaluated by injecting 1µg EN1 protein in 3 months old En1-Het mice.

3 months old En1-Het mice have been injected with either buffer (5 mice) or EN1 (6 mice).

TDP-43 protein levels in motoneurons and cellular localization have been determined with pan-TDP43 antibody at 4.5 months.

Results showed that in En1-Het mice treated by EN1 injection a significant reduction of TDP43 levels in motoneurons is observed (Figure 2).

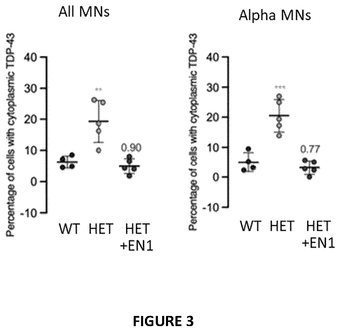

Further results showed a significant reduction of cytoplasmic TDP43 levels (Figure 3).

### 3. Engrailed 1 effect in on phosphorylated TDP-43 levels and localization in alpha-motoneurons.

First, alpha-motoneurons phosphorylated TDP-43 protein levels and cellular localization have been determined using anti-Phospho-TDP43 (Ser409/410) antibody:
(i) in 3-month-old WT mice (5 mice) or
(ii) in 3-month-old *En1-*Het mice (5 mice), or
(iii) in 3-month-old WT mice injected at 1 month with the EN1-neutralizing scFv.

Immunohistochemistry results show :
(i) no staining (nucleus or cytoplasm) in 3-month-old WT mice alpha-motoneurons,
(ii) cytoplasmic staining in 3-month-old *En1-*Het mice alpha-motoneurons (no nuclear staining),
(iii) cytoplasmic staining in alpha-motoneurons of 3-month-old WT mice injected at 1 month with the EN1-neutralizinfg scFv (no nuclear staining).

Then, 2 months old En1-Het mice have been injected with EN1 (6 mice) and phosphorylated TDP-43 protein levels and cellular localization have been determined using anti-Phospho-TDP43 (Ser409/410) antibody at 3 months. Immunohistochemistry results show a return to WT phenotype (no cytoplasmic or nuclear Phospho-TDP43 staining) in alpha motoneurons.

## Claims

1. Engrailed protein, a nucleic acid encoding Engrailed protein or a composition comprising the Engrailed protein or the nucleic acid encoding Engrailed protein, for use in treating or preventing a disease associated with TDP-43 toxicity.

2. The Engrailed protein, the nucleic acid or the composition for use according to claim 1, wherein the disease associated with TDP-43 toxicity is a TDP-43 proteinopathy, preferably selected from the group comprising amyotrophic lateral sclerosis (ALS), frontotemporal lobar degeneration (FTLD), multiple system proteinopathy-A familial disorder in which patients present with ALS, FTLD, inclusion body myositis, Paget's disease of the bone or combinations of these phenotypes (MSP), Alzheimer's disease, dementia with Lewy bodies, Parkinson disease, Huntington disease, cerebral age-related TDP-43 with sclerosis (CARTS), limbic-predominant age-related TDP-43 encephalopathy (LATE), chronic traumatic encephalopathy (CTE), Perry disease, facial onset sensory and motor neuronopathy (FOSMN), sporadic inclusion body myositis (sIBM), primary progressive aphasia (PPA), corticobasal degeneration (CBD), primary progressive aphasia (PPA), hippocampal sclerosis and argyrophilic grain disease (AGD).

3. The Engrailed protein, the nucleic acid or the composition for use according to claim 1 or claim 2, wherein the Engrailed protein decreases cytoplasmic TDP-43 aggregates levels in cells of the nervous system of a subject compared to cytoplasmic TDP-43 aggregates levels in the subject before administration of the Engrailed protein.

4. A method comprising a step of determining the amount of TDP-43 in cells in a biological sample obtained from a subject to determine if an increased level of TDP-43 and/or an increased cytoplasmic accumulation is observed, wherein if an increased level of TDP-43 and/or an increased cytoplasmic accumulation of TDP-43, is observed, the subject can be selected for treatment with a composition comprising Engrailed protein or a nucleic acid encoding Engrailed protein.

5. A method of selecting a subject for treatment with a composition comprising Engrailed protein or a nucleic acid encoding Engrailed protein comprising determining the amount of TDP-43 in cells from a biological sample, particularly in the cytoplasm of cells, and selecting the subject for said treatment.

6. The method according to claim 4 or claim 5, wherein the subject has a familial history of a TDP-43 proteinopathy.

7. The method according to claim 6, wherein the TDP-43 proteinopathy is selected from the group comprising amyotrophic lateral sclerosis (ALS), frontotemporal lobar degeneration (FTLD), multiple system proteinopathy-A familial disorder in which patients present with ALS, FTLD, inclusion body myositis, Paget's disease of the bone or combinations of these phenotypes (MSP), Alzheimer's disease, dementia with Lewy bodies, Parkinson disease, Huntington disease, cerebral age-related TDP-43 with sclerosis (CARTS), limbic-predominant age-related TDP-43 encephalopathy (LATE), chronic traumatic encephalopathy (CTE), Perry disease, facial onset sensory and motor neuronopathy (FOSMN), sporadic inclusion body myositis (sIBM), primary progressive aphasia (PPA), corticobasal degeneration (CBD), primary progressive aphasia (PPA), hippocampal sclerosis and argyrophilic grain disease (AGD).

8. The method according to any one of claims 4 to 7, wherein the biological sample is a cerebrospinal sample.
